# EUROPEAN PATENT SPECIFICATION

(11) **EP 1 462 072 B1**
(45) Date of publication and mention of the grant of the patent: **14.11.2007**
(21) Application number: 04007044.3
(22) Date of filing: 24.03.2004
(51) Int. Cl.: A61F 5/01

(54) **Brace for varus/valgus knee**
Prothese für Varus/Valgus-Knie
Prothèse pour genou varus-valgus

(30) Priority: 27.03.2003 IT VR20030036
(43) Date of publication of application: 29.09.2004
(73) Proprietor: F.G.P. Srl, 37062 Dossobuono (VR) (IT)
(72) Inventor: Turrini, Alberto, 37060 Castel d'Azzano (VR) (IT); Ferrigolo, Moreno, 37062 Dossobuono (VR) (IT)
(74) Representative: Petraz, Gilberto Luigi

(56) References cited:
- FR-A- 2 720 270
- US-A- 4 381 768
- US-A- 4 802 466
- US-A- 4 805 606
- US-A- 5 512 039
- US-A- 5 520 622
- US-A- 6 110 138
- US-B1- 6 436 066

## Description

### TECHNICAL FIELD

This invention concerns a tutor or orthesis for application to a knee affected by varus or valgus type abnormalities.

More in particular, this invention refers to a brace advantageously made from fabric, with a structure such that it can be used to correct axial deformities of the legs.

This invention can be applied in the medical industry with particular reference to manufacturers of prostheses and braces.

### BACKGROUND ART

It is known that valgus and varus are skeletal abnormalities that can occur in axial deformities of the limbs.

The skeletal segments, situated distally to the section in which the deformity is present, are in an abnormal position of abduction in a valgus deformity and of adduction in a varus deformity, in other words in the first case further away from and in the second case closer to the midline of the body.

The most typical case involves the knee which, if varus, tends to bend outwards with respect to the non-deformed axis of the limb.

Vice versa, if the knees are valgus they tend to turn inwards and become closer to each other.

In both of these abnormalities the subject tends to experience greater difficulty in walking the more the obtuse angle is open internally in the case of a varus deformity or open externally in the case of a valgus deformity.

Various solutions for this problem are known to the background art, for example through the use of special knee supports and braces designed to correct the alignment of the knee joint.

While clinically resolving the deformation of the limbs, the solutions proposed to date are not without problems, connected substantially to the use of the orthesis.

One drawback is represented by the fact that these braces are difficult to adjust, particularly when moving around. In fact, a gradual correction of the limb deformity requires continuous measurement of the obtuse angle between the femur and the tibia with a consequent adaptation of the brace, above all as regards the rigid frame.

Another drawback is represented by the fact that such braces are sometimes difficult to put on as they consist of several reciprocally connected parts which can be attached to each other by any appropriate means of temporary blocking.

A further drawback is represented by the fact that the braces usually tend to compress the kneecap area, causing neurological and vascular problems.

Document US-B1-6436066 discloses a truss body joint brace having the features described in the preamble of claim 1 of the present application. This brace is designed to make the knee joint more rigid, to reduce the "specific" load by distributing it over a wider area and, therefore, to prevent injury. According to this solution the distribution of the load is carried out by the tension imparted to the straps and by their geometry. However, this brace is provided of eight different straps, what causes great difficulties to the user in order to correctly adjust the tension of the straps and to modify the pressure exerted on the knee joints.

### DESCRIPTION OF THE INVENTION

This invention proposes to provide a brace for a varus or valgus knee that is able to eliminate or significantly reduce the drawbacks described above.

This invention also proposes to provide a brace for a varus or valgus knee that can be easily produced as well as being reliable and safe.

This is achieved by means of a brace for a varus or valgus knee with the features described in the main claim. The dependent claims described advantageous embodiments of the invention.

The brace for a varus or valgus knee according to the invention comprises a rigid frame equipped with articulated joints in the central part, a fabric panel integral with the frame and presenting an opening in the central kneecap portion and in the rear part, means for constraining the frame to the femur and to the tibia, these means of constraint consisting of a cross-strap hinged to the frame and the fabric panel, these being a single body.

According to the invention, the rigid frame consists of two pairs of uprights, each pair being integral with a respective articulated joint and which can be angularly inclined with respect to the respective articulated joint.

The brace is equipped with means for the continuous adjustment of the obtuse angle formed between the femur and the tibia, these adjustment means consisting of pads which are housed in respective inner pockets positioned in correspondence with each articulated joint and designed to exert pressure on the relative part of the knee joint.

Each pad consists of a deformable toroidal chamber equipped with tubes designed to be connected to any suitable means of pumping for an appropriate fluid. Each tube is easily accessible from the outside and has appropriate means for the input and outlet of the fluid.

Adjusting the pumping pressure ensures a lateral thrust on the knee joint of the subject, causing pressure that acts at the level of the condyles of the knee joint and which is proportional to the deviation from the non-deformed axis of the limb.

In the case of a varus knee the pads should be applied in correspondence with the articulated joints facing outwards, while for cases of valgus knee the pads are housed in the relative pockets adjacent to the inner articulated joints.

The cross-straps acts as tighteners, presenting a device for adjusting the clamping force on the femur and the tibia.

Each of these straps can be fitted with a respective variable extension fixing means, such as for example a piece of Velcro or any appropriate tear fastener.

Each articulated joint can be the type with four angular excursion hinges comprising a platform equipped with at least one pair of hinged couplings for the respective uprights, the platform presenting a shaped central plate and at least one housing for a respective extractable insert designed to come into contact with the plate and at least one longitudinal end of an upright.

The fabric panel can be closed on the front part of the limb to form a tube, being equipped with any appropriate means of temporary fixing such as Velcro fasteners or clip fasteners. The application and adjustment of the brace are therefore particularly easy.

The rigid frame and the articulated joints of the brace are made from lightweight metal alloy or high-resistance composite plastic.

The panel integral with the rigid frame is made from anallergic fabric.

### DESCRIPTION OF THE DRAWINGS

Other features and advantages of the invention are evident in the description which follows of one embodiment of the invention, by way of example and in no way limiting the scope of application, with reference to the accompanying drawing, in which:

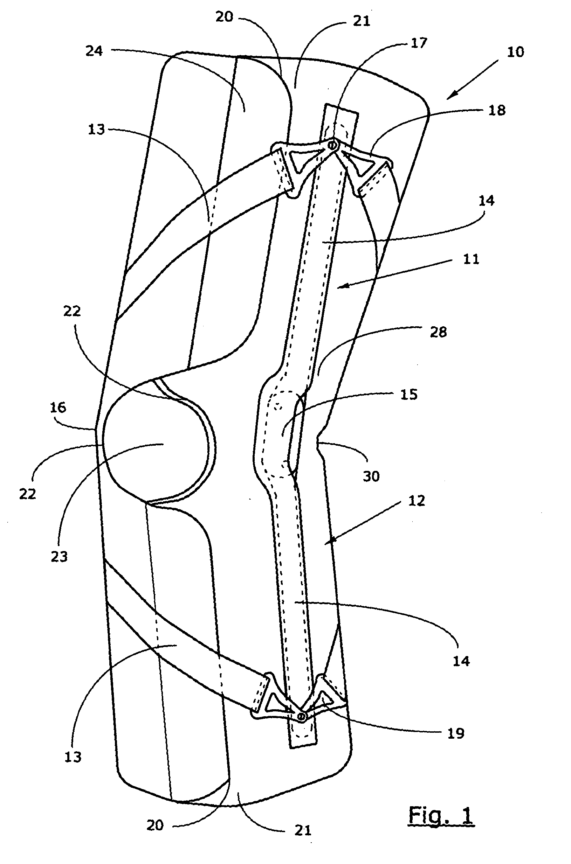

- figure 1 shows a prospective view slightly from above of a brace according to the invention;
- figure 2 shows a prospective view slightly from above of the brace in figure 1 from the opposite side; and
- figure 3 shows an enlarged plan view of a pad.

### DESCRIPTION OF ONE EMBODIMENT

In the figures, the reference number 10 indicates in general a brace, in the case in question a brace 10 for a varus or valgus knee.

The brace 10 comprises a rigid frame 11 to which a panel 12 made from anallergic fabric is fixed, and cross straps 13 which can be hinged to the frame 11 and act as means of constraint to the femur and to the tibia of the user.

The rigid frame 11 consists of two pairs of uprights 14 which in use are positioned at the sides of the limb involved, each pair being integral with a respective articulated joint 15.

Each upright 14 is angularly deformable with respect to the relative articulated joint 15 in order to allow adjustment of the corrective action exerted by the brace 10 on the knee.

Each cross-strap 13 extends from one upright 14 to the opposite one from an attachment position proximal to the knee-cap portion 16 to a position adjacent to the longitudinal end of the brace 10.

The straps 13 are hinged in pairs to the respective uprights 14 presenting an attachment consisting of an eyelet 17 designed to support fork-like elements 18 each of which is connected to the respective strap 13.

Each fork-like element 18 can have an opening 19 to make it lighter in weight.

The straps 13 can be equipped with means for adjusting their length so that they can be used as tighteners to ensure an adequate grip of both the tibial and the femoral parts.

The means for adjusting the cross-straps 13 can consist of Velcro fasteners or clip fasteners (not shown in the drawings).

The panel 12 consists of a single wrap-around elastic piece presenting a first longitudinal flap 20 surmounting a second flap 21 forming a tubular shape suitable to accommodate the limb to be corrected and has a hole 30 which when fitted is positioned at the rear of the knee joint.

The central part of each flap 20, 21 presents a recess 22 forming an opening 23 designed to accommodate the user's kneecap.

The first flap 20 can be fixed to the second flap 21 by any suitable means of constraint, for example by Velcro fasteners 24 or clip fasteners (not shown in the drawings).

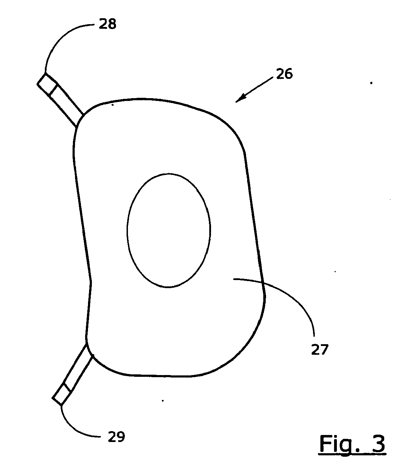

The inner part of the panel 12 adjacent to each articulated joint 15 is equipped with a pocket 25 designed to accommodate a respective pad 26 (figure 3).

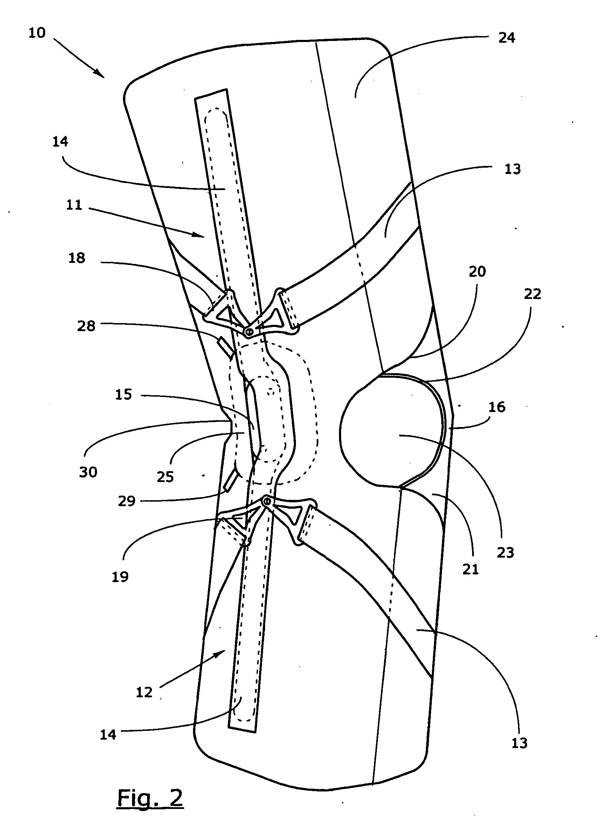

The pad 26 consists of a deformable toroidal chamber 27 equipped with tubes 28 designed to be connected to any suitable pumping means for an appropriate fluid. Each tube 28 is easily accessible from the outside when in place (figure 2) and has a respective closing system 29.

The rigid frame 11 and the articulated joints 15 of the brace 10 can be made from lightweight metal alloy, for example aluminium, or from high-resistance composite plastic.

The presence of the Velcro fasteners 24 ensures easy opening of the brace 10 for rapid removal from the limb, making it easy to use and to fit.

The presence of the pads 26 allows methodical and continuous adjustment of the obtuse angle between the femur and the tibia thanks to the inflation pressure of the toroidal chamber 27 designed to exert a pressing action at the sides of the knee joint.

This adjustment is extremely quick as it can be carried out without having to open the brace 10.

## Claims

1. A brace (10), for a varus or valgus knee, which comprises a rigid frame (11) equipped in its central part with articulated joints (15), said rigid frame consisting of two respective pairs of uprights (14), each pair of uprights being integral with a respective one of said articulated joint (15) located in a central part of the frame between the two uprights, a fabric panel (12) integral with the frame (11) and when in place presenting an opening (23) at the front of the limb, further comprising means of constraint of the frame (11) to the femur and the tibia consisting of cross-straps (13) hinged to the frame (11), **characterised in that** it comprises means for the continuous adjustment of the obtuse angle formed between the femur and the tibia, said adjustment means consisting of inflatable pads (26) which are respectively accommodated into inner pockets (25) of the panel (12) and positioned in correspondence with each respective articulated joint (15), said pads being inflated in such a way as to exert a predetermined pressing action at the sides of the knee joint and thereby adjusting the obtuse angle formed between the femur and the tibia.

2. A brace (10)according to claim 1, **characterised in that** the fabric panel (12) consists of a single piece.

3. A brace (10) according to any one of the preceding claims, **characterised in that** the opening and closing of the brace (10) is at the front of the limb by fixing a first flap (20) to a second flap (21).

4. A brace (10) according to any one of the preceding claims, **characterised in that** each pad (26) consists of a deformable toroidal chamber (27) equipped with tubes (28) easily accessible when in place from outside the brace (10).

5. A brace (10) according to claim 4, **characterised in that** each tube (28) is equipped with a respective closing system (29).

6. A brace (10) according to any one of the preceding claims, **characterised in that** each upright (14) can be angularly inclined with respect to the corresponding articulated joint (15).

7. A brace (10) according to any one of the preceding claims, **characterised in that** the longitudinal flaps (20, 21) of the panel (12) are equipped with temporary fixing means.

8. A brace (10) according to claim 7, **characterised in that** the temporary fixing means consist of velcro fasteners and/or of clip fasteners.

9. A brace (10) according to any one of the preceding claims, **characterised in that** each cross-strap (13) is equipped with a respective fixing means of variable length.

10. A brace (10) according to claim 9, **characterised in that** the variable length fixing means consist of a velcro fastener and/or of a clip fastener.

11. A brace (10) according to any one of the preceding claims, **characterised in that** the rigid frame (11) and the articulated joints (15) are made from lightweight metal alloy.

12. A brace (10) according to any one of the preceding claims, **characterised in that** the rigid frame (11) and the articulated joints (15) are made from high-resistance composite plastic.

13. A brace (10) according to any one of the preceding claims, **characterised in that** the panel (12) is made from anallergic fabric.

## Patentansprüche

1. Schiene (10) für ein Knie in Varus- oder Valgusstellung, bestehend aus einem festen Rahmen (11), der in seinem mittleren Bereich gegliederte Scharniere (15) aufweist, wobei der Rahmen aus jeweils zwei Vertikalteilpaaren (14) besteht und wobei jedes Vertikalteilpaar mit je einem gegliederten Scharnier (15) integriert ist, das sich in einem mittleren Teil des Rahmens zwischen zwei Vertikalteilen befindet,
aus einer mit dem Rahmen (11) integrierten Stoffbahn (12), die - wenn angelegt - am Vorderteil des Gliedes eine Öffnung (23) aufweist, und
aus einer Vorrichtung, die den Rahmen (11) am Oberschenkelknochen und am Schienbein festhält und aus Quergurten (13) besteht, die am Rahmen (11) angelenkt sind,
**dadurch gekennzeichnet, dass** sie eine Vorrichtung für die laufende Einstellung des zwischen Oberschenkelknochen und Schienbein gebildeten stumpfen Winkels enthält, wobei die Einstellung aus aufblasbaren Kissen (26) besteht, die jeweils in den Innentaschen (25) der Stoffbahn (12) untergebracht sind und jeweils in Bezug auf jedes gegliederte Scharnier (15) positioniert sind, wobei die Kissen so aufgeblasen werden, dass sie einen vorbestimmten Druck auf die Seiten des Kniegelenks ausüben und so den zwischen Oberschenkelknochen und Schienbein gebildeten stumpfen Winkel einstellen.

2. Schiene (10) gemäß Anspruch 1, **dadurch gekennzeichnet, dass** die Stoffbahn (12) aus einem einzigen Stück besteht.

3. Schiene (10) gemäß einem der vorhergehenden Ansprüche, **dadurch gekennzeichnet, dass** die Schiene (10) vorn am Glied geöffnet und geschlossen wird, indem eine Lasche (20) an einer zweiten Lasche (21) befestigt wird.

4. Schiene (10) gemäß einem der vorhergehenden Ansprüche, **dadurch gekennzeichnet, dass** jedes Kissen (26) aus einer verformbaren ringförmigen Kammer (27) besteht, die mit Schläuchen (28) versehen ist, welche bei angelegter Schiene leicht von außerhalb der Schiene (10) zugänglich sind,

5. Schiene (10) gemäß Anspruch 4, **dadurch gekennzeichnet, dass** jeder Schlauch (28) mit einem Verschlusssystem (29) versehen ist.

6. Schiene (10) gemäß einem der vorhergehenden Ansprüche, **dadurch gekennzeichnet, dass** jedes Vertikalteil (14) relativ zum entsprechenden gegliederten Scharnier (15) winkelig geneigt werden kann.

7. Schiene (10) gemäß einem der vorhergehenden Ansprüche, **dadurch gekennzeichnet, dass** die länglichen Laschen (20, 21) der Stoffbahn (12) mit provisorischen Befestigungsmitteln versehen sind.

8. Schiene (10) gemäß Anspruch 7, **dadurch gekennzeichnet, dass** die provisorischen Befestigungsmittel aus Klett- und/oder Clipverschlüssen bestehen.

9. Schiene (10) gemäß einem der vorhergehenden Ansprüche, **dadurch gekennzeichnet, dass** jeder Quergurt (13) mit entsprechenden Befestigungsmitteln veränderlicher Länge versehen ist.

10. Schiene (10) gemäß Anspruch 9, **dadurch gekennzeichnet, dass** die Befestigungsmittel von veränderlicher Länge aus einem Klett- und/oder Clipverschluss bestehen.

11. Schiene (10) gemäß einem der vorhergehenden Ansprüche, **dadurch gekennzeichnet, dass** der feste Rahmen (11) und die gegliederten Scharniere (15) aus einer leichten Metalllegierung bestehen.

12. Schiene (10) gemäß einem der vorhergehenden Ansprüche, **dadurch gekennzeichnet, dass** der feste Rahmen (11) und die gegliederten Scharniere (15) aus hoch-widerstandsfähigem Plastikverbundstoff bestehen.

13. Schiene (10) gemäß einem der vorhergehenden Ansprüche, **dadurch gekennzeichnet, dass** die Stoffbahn (12) aus anallergischem Gewebe hergestellt ist.

## Revendications

1. Genouillère (10), pour un genou varus ou valgus, comportant une structure rigide (11), équipée à sa partie centrale de jointures articulées (15), ladite structure rigide comprenant deux paires de montants (14), chaque paire de montants étant respectivement solidaire d'une desdites jointures articulées (15) correspondantes, placées au niveau de la partie centrale de la structure entre les deux montants, un panneau (12) de tissu solidaire de la structure (11) présentant une ouverture (23) à l'avant du membre lorsqu'il est mis en place, genouillère comportant de plus des moyens de serrage de la structure (11) contre le fémur et le tibia, lesdits moyens de serrage étant des sangles croisées (13) articulées sur la structure (11), genouillère **caractérisée en ce qu'**elle comporte des moyens d'ajustement de l'angle obtus formé par le fémur et le tibia, lesdits moyens d'ajustement étant des coussins (26) gonflables, qui sont respectivement placés dans des poches intérieures (25) du panneau (12) et placés en correspondance avec chaque jointure articulée (15) correspondante, lesdits coussins étant gonflés de manière à exercer une pression prédéterminée sur le côté de l'articulation du genou et ainsi ajustant l'angle obtus formé par le fémur et le tibia.

2. Genouillère (10) selon la revendication 1, **caractérisée en ce que** le panneau (12) est réalisé en une seule pièce.

3. Genouillère (10) selon l'une quelconque des revendications précédentes, **caractérisée en ce que** l'ouverture et la fermeture de la genouillère (10) est réalisée sur l'avant du membre en fixant un premier rabat (20) à un second rabat (21).

4. Genouillère (10) selon l'une quelconque des revendications précédentes, **caractérisée en ce que** chaque coussin (26) est une chambre toroïdale (27) déformable équipée de tubes (28), facilement accessibles de l'extérieur de la genouillère (10) quand les coussins (26) sont mis en place.

5. Genouillère (10) selon la revendication 4, **caractérisée en ce que** chaque tube (28) est équipé d'un mécanisme de fermeture (29) correspondant.

6. Genouillère (10) selon l'une quelconque des revendications précédentes, **caractérisée en ce que** chaque montant (14) peut être incliné angulairement par rapport à la jointure articulée (15) correspondante.

7. Genouillère (10) selon l'une quelconque des revendications précédente, **caractérisée en ce que** les rabats (20,21) longitudinaux du panneau (12) sont équipés de moyens de fixation temporaire.

8. Genouillère (10) selon la revendication 7, **caractérisée en ce que** les moyens de fixation temporaire sont des attaches de type velcro et/ou des attaches en forme de clip.

9. Genouillère (10) selon l'une quelconque des revendications précédentes, **caractérisée en ce que** chaque sangle croisée (13) est équipée de moyens de fixation correspondant de longueur variable.

10. Genouillère (10) selon la revendication 9, **caractérisée en ce que** les moyens de fixation de longueur variable sont des attaches de type velcro et/ou des attaches en forme de clip.

11. Genouillère (10) selon l'une quelconque des revendications précédentes, **caractérisée en ce que** la structure rigide (11) et les jointures articulées (15) sont réalisées en alliage métallique léger.

12. Genouillère (10) selon l'une quelconque des revendications précédentes, **caractérisée en ce que** la structure rigide (11) et les jointures articulées (15) sont réalisées en plastique composite hautement résistant.

13. Genouillère (10) selon l'une quelconque des revendications précédentes, **caractérisée en ce que** le panneau (12) est réalisé dans un tissu non allergisant.
